# EUROPEAN PATENT APPLICATION

(11) **EP 4 461 301 A1**
(43) Date of publication of application: **13.11.2024**
(21) Application number: 23171968.3
(22) Date of filing: 07.05.2023
(51) Int. Cl.: A61K 35/19, A61K 48/00, C12N 5/078, C12N 5/10

(54) **A METHOD FOR PRODUCING ENGINEERED PLATELETS**

(71) Applicant: HemostOD SA, 1025 Saint-Sulpice (CH)
(72) Inventor: Burnier, Laurent, 1006 Lausanne (CH); Humbert, Magali, 1680 Romont (CH); Rocca, Céline, 1024 Ecublens (CH)
(74) Representative: DTS Patent- und Rechtsanwälte PartmbB

(57) **Abstract**

The present invention relates to a method for producing engineered platelets, the method comprising at least the following steps:
- transduction of a therapeutic sequence into progenitors of megakaryocytes, CD34-expressing hematopoietic stem and progenitor cells, immature megakaryocytes or megakaryocytes, using a DNA-based vector, the therapeutic sequence encoding at least one therapeutic product,
- generating platelets from mature megakaryocytes derived from the transduced progenitors of megakaryocytes or from the transduced CD34-expressing hematopoietic stem and progenitor cells or from the transduced immature megakaryocytes or from the transduced megakaryocytes,
wherein the platelets contain the at least one therapeutic product.

Further, the invention relates to the use of an engineered platelet or a pharmaceutical preparation comprising engineered platelets expressing at least one therapeutic coding sequence and/or at least one coding sequence for a receptor protein, for use in medicine, in particular for the treatment of at least one of cancer, acute inflammation, chronic inflammation, bleeding, coagulation, liver disease.

## Description

The present invention relates to a method for producing engineered platelets.

Further, the present invention relates to the use of a pharmaceutical preparation comprising engineered platelets or the use of engineered platelets in a method for the treatment of a disease.

Still further, the present invention relates to the use of a pharmaceutical preparation comprising engineered platelets or the use of engineered platelets in a method for the treatment of cancer, acute inflammation, chronic inflammation, bleeding and/or coagulation disorders, liver disease.

Delivery of pharmaceutical compounds (also referred to as therapeutic compounds or therapeutic products) to a specific cell or tissue or organ in the body of a patient is until now a challenge. Plasmids, antisense oligonucleotides (ASO), and new drug modalities such as small interfering RNA (siRNA) and microRNA (miRNA), gene-editing guide RNA (gRNA) have been recently shown to potentially be used to treat many pathological conditions, but still face the challenge of delivery. This is also true for other classes of therapeutics, such as monoclonal antibodies (mAbs). Besides the challenge of administration, half-life in plasma, and proper distribution to the pharmacological target, these compounds are biologically active, therefore limiting the therapeutic dosage to avoid toxicity and off-target effects. New drug delivery systems, such as lipid nanoparticles or exosomes, suffer from the same problems, intrinsic toxicity, systemic distribution, and manufacturing challenges. Therefore, safe drug delivery is still a challenge, and with the emergence of new drug modalities and new classes of targets, safe tissue-specific delivery is a must.

Platelets, small, nucleated cells that circulate in the blood and are involved in hemostasis, have been proposed as vehicles for delivering therapeutics to the site of interest in disease.

Indeed, platelets deliver their cargo, contained in cellular granules, only when activated. Platelets contain three major types of granules, alpha-granules, dense granules, and lysosomes. Each granule contains distinct cargo, with different biogenesis. Moreover, platelets can also generate microparticles upon strong activation (Burnier et al., 2009). These microparticles are or comprise exosomes and ectosomes, and differ by their cargo and mechanism of formation. Altogether, platelets naturally possess the full machinery to be activated at very specific sites and moments, such as during inflammation or bleeding, and can immediately deliver their cargo.

Thus, using platelets to deliver therapeutics is elegant and allows for a controlled release of therapeutics. Indeed, this approach, so far, requires a passive loading of the therapeutics inside or at the surface of platelets. This requires the development of specific cell culture media and process to induce or force loading, jeopardizing the quality of both the loaded therapeutic and the platelets. The cargo may also then be loaded into a cellular compartment that will not be released upon platelet activation.

WO2020/006539A1 discloses methods for preparing drug-loaded platelets, wherein platelets are treated with a drug and with a loading buffer. This means, that platelets are passively loaded.

It is an object of the present invention to provide a method for producing platelets, in particular engineered platelets, as a safe vehicle for at least one therapeutic compound, in particular into specific cells and/or tissues, while protecting the active therapeutic compound of degradation or inhibition, while avoiding passive loading of the platelets.

The object is solved according to the present invention by a method, with the features of claim 1, accordingly, a method for producing engineered platelets, the method comprising at least the following steps:
- transduction of a therapeutic sequence into progenitors of megakaryocytes, CD34-expressing hematopoietic stem and progenitor cells (HSPC), immature megakaryocytes or megakaryocytes, using a DNA-based vector, the therapeutic sequence encoding at least one therapeutic product,
- generating platelets from mature megakaryocytes derived from the transduced progenitors of megakaryocytes or from the transduced CD34-expressing HSPC or from the transduced immature megakaryocytes or from the transduced megakaryocytes,
wherein the platelets contain the at least one therapeutic product.

The invention is based on the basic idea that megakaryocytes (MKs) (e.g. immortalized MKs), progenitors of megakaryocytes (e.g. immortalized progenitors of megakaryocytes), or CD34-expressing HSPC (e.g. immortalized CD34-expressing HSPC), are engineered to express at least one therapeutic sequence and/or therapeutic product. Engineered cells can be then differentiated into mature MKs and manipulated to generate platelets. The resulting platelets express the respective at least one therapeutic product and can be further used for therapeutic approaches, e.g. as or in pharmaceutical compositions. In other words, passive loading of the therapeutic compound inside or at the surface of mature platelets is avoided. The method is therefore simple (compared to loading platelets) and allows the production of correspondingly engineered plates on a large scale, at comparatively low cost.

After infusion into patients, respective engineered platelets will circulate in blood, in a resting state, tightly controlled by plasmatic and cellular components of blood vessels. Inside circulating engineered platelets, the at least one therapeutic product encoded by the therapeutic sequence is shielded from inhibition or degradation. Only after arrest at a specific platelet activation site, such as bleeding, injury, cancer, or inflammation, platelets will be activated, and release their cargo, including the at last one therapeutic product. Thus, engineered platelets produced by the method according to the invention can be regarded as pathological process-targeted therapeutic product delivery vehicles.

For instance, lentiviruses can be used for transduction of an expression cassette into cells. In particular, the expression cassette can comprise a promoter, or regulatory sequence. It can be a promoter for high level of expression, such as spleen focus-forming virus (SFFV), or with low level of expression, or with a regulated level of expression, or with a megakaryocytic-lineage specific promoter. Next to the promoter, one or multiple coding sequences for the therapeutic product, in particular also referred to as one or multiple therapeutic sequences can be added. In particular, the expression cassette can be referred to as therapeutic product expression cassette. Also, multiple viruses can be used to express multiple therapeutic products.

In particular, the therapeutic sequence can encode for at least one of messenger RNA (mRNA), small interfering RNA (siRNA), short hairpin RNA (shRNA) or micro RNA (miRNA).

ShRNA is an artificial molecule with a tight hairpin turn that can be used to silence target gene expression via RNA interference.

SiRNA is a class of double-stranded RNA at first non-coding RNA molecules, typically 20-24 (normally 21) base pairs in length, similar to miRNA, and operating within the RNA interference (RNAi) pathway. It interferes with the expression of specific genes with complementary nucleotide sequences by degrading mRNA after transcription, preventing translation.

A microRNA is a small single-stranded non-coding RNA molecule (containing about 22 nucleotides) found in plants, animals and some viruses, that functions in RNA silencing and post-transcriptional regulation of gene expression.

MiRNAs function via base-pairing with complementary sequences within mRNA molecules. As a result, these mRNA molecules are silenced, by one or more of the following processes: (1) cleavage of the mRNA strand into two pieces, (2) destabilization of the mRNA through shortening of its poly(A) tail, and (3) less efficient translation of the mRNA into proteins by ribosomes.

Thus, the engineered platelets obtained by the method can allow silencing a gene of interest (via miRNA, siRNA, shRNA) in a cell targeted. Further, mRNA is a single-stranded molecule of RNA that corresponds to the genetic sequence of a gene, and is read by a ribosome in the process of synthesizing a protein. mRNA can encode for e.g. an antibody, a cytokine, a receptor protein, or chimeric receptor protein, etc. Thus, the engineered platelets obtained by the method can provide an antibody and/or cytokine for a desired reaction.

In general, the therapeutic sequence can be single. Thus, the sequence for the therapeutic compound appears only once. Alternatively, the therapeutic sequence can be tandem for multiplexing. This means, that copies of sequences lie adjacent to each other in the same orientation (direct tandem repeats) or in the opposite direction to each other (inverted tandem repeats).

In particular, the therapeutic product expression cassette can be a DNA sequence.

In particular, the therapeutic product can be a miRNA and/or siRNA against podoplanin (PDPN). Thus, platelets releasing miRNA and/or siRNA against PDPN, can be produced with the method according to the present invention, to decrease the expression of PDPN in tissue or organs, e.g. cancer tissue.

Examples of miRNA that can target PDPN are miR-29b and miR-125a (Cortez et al., 2010) (mature sequences are GCUGGUUUCAUAUGGUGGUUUAGA (SEQ ID NO: 1) and UCCCUGAGACCCUUUAACCUGUGA (SEQ ID NO: 2), respectively).

In particular, the coding human sequence (5'-3', stemloop-forming precursor RNAs) for miR-29b is CUUCAGGAAGCUGGUUUCAUAUGGUGGUUUAGAUUUAAAUAGUGAUUGUCUAGCACC AUUUGAAAUCAGUGUUCUUGGGGG (SEQ ID NO: 4), and for miR-125a is UGCCAGUCUCUAGGUCCCUGAGACCCUUUAACCUGUGAGGACAUCCAGGGUCACAG GUGAGGUUCUUGGGAGCCUGGCGUCUGGCC (SEQ ID NO: 5), (source: https://www.mirbase.org/).

An example of siRNA sequence (21-nt) is ACAAAACGGUGUCAUUUGCAC (SEQ ID NO: 3), (Naito et al., 2009).

PDPN is a mucin-type protein with a mass of 36- to 43-kDa, also known as gp38, T1α, D2-40, Glycoprotein 36, or Aggrus. It is relatively well conserved between species, with homologues in humans, mice, rats, dogs and hamsters. This gene encodes a type-I, integral membrane, heavily O-glycosylated glycoprotein with diverse distribution in human tissues. The physiological function of this protein may be related to its mucin-type character. This protein has been found to have functions in lung alveolar cells, kidney podocytes, and lymphatic endothelial cells. More recently, this protein has been found in neural tissue in both mouse and human samples. In lymphatic endothelial cells, experimentation has indicated that PDPN plays a role in proper formation of linkages between the cardiovascular system and the lymphatic systems, typically causing fatty liver disease in these mice. Although the exact function is unknown in many tissues, PDPN is generally receptive to detection via immunofluorescent staining and has been shown to co-localize with the protein nestin, a type VI intermediate filament protein expressed almost primarily in neural tissues.

PDPN has been studied extensively in the cancer field. It is a specific lymphatic vessel marker, and since lymphangiogenesis levels are correlated with poor prognosis in cancer patients, it can be used as a diagnostic marker. It is often upregulated in certain types of cancer, including several types of squamous cell carcinomas, malignant mesothelioma and brain tumors. Moreover, it can be upregulated by cancer-associated fibroblasts (CAFs) in the tumor stroma, where it has been associated with poor prognosis (Shindo et al., 2013). In squamous cell carcinomas, PDPN is believed to play a key role in the cancer cell invasiveness by controlling invadopodia, and thus mediating efficient extracellular matrix degradation (Martin-Villar et al., 2015).

Thus, PDPN is overexpressed in tumor cells and surrounding tissues. Megakaryocytes, or progenitor of megakaryocytes, or stem cells, in particular CD34-expressing HSPC, can be transduced with a vector (e.g. virus, e.g. lentivirus) comprising an expression cassette (and/or therapeutic sequence), to express anti-PDPN miRNA and/or siRNA. Platelets can be generated from these cells after differentiation into mature megakaryocytes and infused into patients with PDPN-positive tumors.

Platelets naturally express C-type lectin domain family 1 member B (CLEC-2), the receptor for PDPN. Engineered platelets are therefore naturally activated in tissue overexpressing PDPN, such as in PDPN-positive cancer. PDPN overexpression in cancer usually leads to increased metastasis and prothrombotic events.

Thus, immediately after activation, engineered platelets will liberate miRNA and/or siRNA against PDPN in the vicinity of tumors, possibly via microparticles. SiRNA or miRNA will be engulfed or endocytosed by surrounding cells, and their PDPN expression will be downregulated. Localized downregulation can decrease tumor progression and metastasis.

In particular, in healthy tissue, the expression of PDPN does not lead to platelet activation - despite expression of PDPN and CLEC-2. In particular, this is due to the nature of the vasculature, preventing platelet activation. Moreover, platelets do not circulate in lymphatic vessels. However, in PDPN-overexpressing cancer, platelets are activated (supported by literature). In patients with infused engineered platelets according to the present invention, platelets will be resting until being in the tumor vicinity, leading to the release of anti-PDPN products by platelets.

It is a second object of the present invention to deliver therapeutics as close as possible to target cells and/or tissues and/or organs, while avoiding therapeutics to be active in healthy cells and/or tissues and/or organs or degraded while in blood circulation.

Thus, the expression cassette, delivered by the DNA-based vector, can further (or alternatively) comprise at least one coding sequence for a receptor protein, e.g. a cell or tissue or organ specific receptor protein, wherein the platelets express the receptor protein (in particular the targeting moiety). In other words, the DNA-based vector can further comprise at least one coding sequence for a receptor protein, e.g. a cell or tissue or organ specific receptor protein, wherein the platelets express the receptor protein. This will allow platelets, expressing the receptor protein, to be activated when the ligand is present in the surrounding platelet environment, for precise activation of platelets at a site of interest.

Indeed, for some indications, natural mechanisms of activation may not be sufficient to 1) trigger sufficient activation for cargo release, 2) homing at site of interest. The present invention also provides a solution to generate platelets capable of targeting specific cells and/ or tissues and/or organs that are not the natural place of platelets activation.

In general, the receptor protein can be a non-modified receptor protein (in particular a non-modified cellular receptor protein), a receptor protein complex (in particular a cellular receptor complex), a chimeric receptor protein (in particular a cellular chimeric receptor protein), or a chimeric receptor protein containing a gain-of-function mutation.

A receptor complex is composed of at least one core receptor protein and receptor-associated proteins, which have profound effects on the overall receptor structure, function and localization. A chimeric receptor can be defined as a special receptor created in the laboratory that is designed to bind to certain proteins on cancer cells.

In particular, HSPC, in particular immortalized HSPC, or progenitors of megakaryocytes (MKs), in particular immortalized progenitors of megakaryocytes (MKs), or MKs, in particular immortalized MKs can be additionally or alternatively engineered to express at least one targeting moiety. Only after arrest at a specific site, such as bleeding, injury, cancer, or inflammation, or at a site expressing the ligand of the transgenic receptor expressed by engineered platelets, platelets will be activated, and release their cargo, including the at least one therapeutic compound. The invention therefore can lead to a tissue-targeted therapeutic compound delivery method and can be considered a multispecific drug. Platelets can be activated by multiple, redundant pathways; therefore, a chimeric receptor can increase the specificity of the activation, being part of the surface activation matrix. Overall, only a net intra-platelet signaling in favor of activation will trigger full activation and microparticle release, decreasing the risk of unspecific release of cargo. In other words, the method can allow the production of platelets expressing a tissue-specific receptor that will facilitate platelet arrest at site of interest, and trigger or potentiate cargo release.

In particular, the coding sequence for the receptor protein can comprise mRNA.

In particular, the at least one coding sequence for the receptor protein can encode for, but not limited to, a single-chain variable fragment (scFv) protein, a receptor ligand, a full receptor protein or an extracellular protein domain of a receptor, a nanobody, a designed ankyrin repeat proteins (DARPins), or adapter chimeric antigen receptor (adCAR), together with a transmembrane domain or anchoring region such as a GPI-anchor. This essential or minimal protein coding sequence can be further extended to code for cytoplasmic signaling domains, in order to initiate signaling and platelet activation. It can be achieved by expressing a chimeric receptor, as follows: the extracellular domain contains the tissue specific moiety (e.g. scFv, or DARPin), conjugated to a transmembrane domain and a CLEC-2 intracellular domain. Upon binding to the target of the receptor and its dimerization due to clustering, followed by CLEC-2 phosphorylation by platelet Syk tyrosine kinase, signaling pathway will activate platelets allowing precise activation of platelets at a site of interest. To enhance signaling, a gain-of-function mutation can be introduced in the coding sequence.

One example of targeting moiety that can be expressed at the surface of engineered platelets is a scFv targeting PDPN, to increase homing of therapeutic platelets to the tumor environment, where CLEC-2 will lead to platelet activation and release of therapeutics.

Another example is to take advantage of the overexpression of transferrin receptor by tumor cells (Gatter et al., 1983). Therefore, expressing transferrin, or more elegantly a scFv binding to transferrin on engineered platelets will also improve homing of platelets to the tumor environment. The same strategy can be used by targeting the folate receptor (FR), also expressed in many cancers (Sudimack & Lee, 2000). Finally, all monoclonal antibodies (mAb) targeting cancer cells, such as EGFR (e.g. panitumumab) could be used, by generating scFv from the Fab domain of the mAbs using current molecular biology methods.

Another example is the expression of an adCAR on platelets. An adCAR is a chimeric receptor binding specifically to another targeting protein (or targeting module), such as a monoclonal Antibody (mAb).

A therapeutic mAb is conjugated to a biotin or another epitope tag (to form a linker-label epitope (LLE)). Therapeutic mAb is dosed or infused according to its pharmacokinetic and distribution parameters. At different time points during treatment, engineered platelets expressing an adCAR will be infused. Platelets will then have a specific homing behavior, towards tissues expressing the target of the mAb. Upon arrest and full activation, platelet will release their therapeutic cargo.

Alternatively, the coding sequence could encode for a radio-sensitizer, to increase sensitivity of platelet-targeted tissue for radiotherapy. Indeed, some miRNA such as miR-621, targeting SETDB1 in hepatocellular carcinoma, can act as radiosensitizer (Shao et al., 2019). Similarly, a short siRNA against S100A4 enhances the radiosensitivity of human adenocarcinomic epithelial cells (Qi et al., 2016).

In general, the platelets can be produced for therapeutic use.

The invention further relates to the use of a pharmaceutical preparation comprising engineered platelets expressing at least one therapeutic sequence and/or at least one coding sequence for a receptor protein, such as e.g. a cell or tissue or organ specific receptor protein, in a method for the treatment of a disease. For instance, the pharmaceutical preparation could comprise engineered platelets expressing siRNA and/or miRNA and/or shRNA against PDPN. After administration of the pharmaceutical composition, engineered platelets will be activated in tissue overexpressing PDPN, such as in many cancers, since platelets express CLEC-2, the receptor of PDPN. Immediately after binding to CLEC-2, platelets could liberate miRNA or siRNA against PDPN in the vicinity by microparticles (exosomes and ectosomes). These microparticles could be engulfed by surrounding cells, and their PDPN expression could be downregulated. Localized downregulation of PDPN could for instance decrease tumor progression and metastasis. Additionally, and/or alternatively, the pharmaceutical preparation could comprise engineered platelets expressing at least one coding sequence for a receptor protein. For instance, the protein could be a scFv derived from a tissue-targeting mAb.

In particular, the invention further relates to the use of a pharmaceutical preparation comprising engineered platelets expressing at least one therapeutic sequence and/or at least one coding sequence for a receptor protein, such as e.g. a cell or tissue or organ specific receptor protein, in a method for the treatment of at least one of cancer, acute inflammation, chronic inflammation, bleeding, coagulation, liver disease. It is known that platelets are normally and/or naturally activated in these disease conditions. Also, the invention relates to the use of engineered platelets expressing at least one therapeutic sequence and/or at least one coding sequence for a receptor protein such as e.g. a cell or tissue or organ specific receptor protein, in a method for the treatment of a disease. For instance, the engineered platelets express siRNA and/or miRNA and/or shRNA against PDPN. After administration of the engineered platelets, engineered platelets can be activated in tissue overexpressing PDPN, because platelets express CLEC-2, a PDPN receptor. Immediately after binding to CLEC-2, platelets can liberate miRNA or siRNA against PDPN in the vicinity by microparticles (exosomes and ectosomes). These microparticles can be engulfed by surrounding cells, and their PDPN expression can be downregulated. Localized downregulation of PDPN can for instance decrease tumor progression and metastasis (in particular, platelets will only be activated where PDPN is highly expressed, because in healthy tissue the balance towards platelets inhibition is favorable). Additionally, and/or alternatively, the pharmaceutical preparation can comprise engineered platelets expressing at least one coding sequence for a receptor protein. For instance, the receptor protein could be a scFv derived from a tissue-targeting mAb, or a scFv with signaling intracellular domain (CAR or adCAR).

Also, the invention relates to the use of engineered platelets expressing at least one therapeutic sequence and/or at least one coding sequence for a receptor protein, such as e.g. a cell or tissue or organ specific receptor protein, in a method for the treatment of at least one of cancer, acute inflammation, chronic inflammation, bleeding, coagulation, liver disease.

Further, the invention relates to a pharmaceutical preparation comprising engineered platelets expressing at least one a therapeutic sequence and/or at least one coding sequence for a receptor protein, such as e.g. a cell or tissue or organ specific receptor protein, for use in medicine.

Further, the invention relates to a pharmaceutical preparation comprising engineered platelets expressing at least one therapeutic sequence and/or at least one coding sequence for a receptor protein, such as e.g. a cell or tissue or organ specific receptor protein, for use in a method for the treatment of at least one of cancer, acute inflammation, chronic inflammation, bleeding, coagulation, liver disease.

Further the invention relates to an engineered platelet expressing at least one therapeutic coding sequence and/or at least one coding sequence for a receptor protein, such as e.g. a cell or tissue or organ specific receptor protein, for use in medicine.

Also, the invention relates to an engineered platelet expressing at least one therapeutic sequence and/or at least one coding sequence for a receptor protein, such as e.g. a cell or tissue or organ specific receptor protein, for use in a method for the treatment of at least one of cancer, acute inflammation, chronic inflammation, bleeding, coagulation, liver disease.

Further details and advantages of the present invention shall now be disclosed in the connection with the drawings.

It is shown in
- **Fig. 1**: a diagram of the method for producing engineered platelets according to the present invention;
- **Fig. 2**: a schematic illustration of how genetic modification of an immortalized megakaryocyte leads to the production of an engineered platelet;
- **Fig. 3**: an illustration of the release of microparticles from platelets;
- **Fig. 4**: a schematic view of engineered platelets releasing a therapeutic compound at a site of interest;
- **Fig. 5**: an example of a lentiviral transfer plasmid with an expression cassette comprising a therapeutic coding sequence;
- **Fig. 6**: a schematic view of the mechanism leading to the therapeutic utility of platelets expressing CAR or adCAR;
- **Fig. 7**: an example of a miRNA sequence, introduced into megakaryocyte progenitor, targeting B2M;
- **Fig 8**: an example of CAR expressed in megakaryocyte progenitors and mature megakaryocytes; and
- **Fig 9**: an example of shRNA expressed in platelets and megakaryocytes after transduction of CD34⁺ cells.

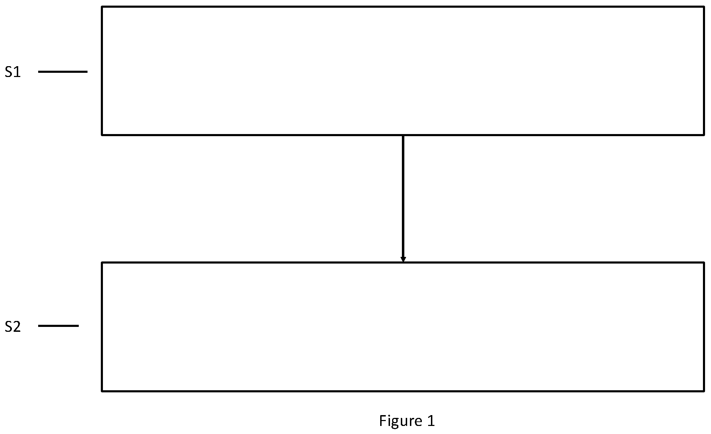

**Fig. 1** shows a diagram of the method for producing engineered platelets according to the present invention.

In this embodiment, the method comprises two steps. However, the method could also comprise more than two steps.

In a first step S1, a therapeutic sequence is transduced into progenitors of megakaryocytes, CD34-expressing HSPC, immature megakaryocytes or megakaryocytes, using a DNA-based vector, the therapeutic sequence encoding at least one therapeutic product.

In this embodiment, a lentivirus is used to transduce an expression cassette into the cells. In particular, the expression cassette comprises a promoter. Next to the promoter, a coding sequences for the therapeutic compound is added.

In an alternative embodiment, the expression cassette is transduced into immature megakaryocytes.

Alternatively, mature megakaryocytes or any progenitors of megakaryocytes could be transduced.

In this embodiment, the expression cassette comprises a therapeutic sequence.

In an alternative embodiment, the expression cassette comprises more than one therapeutic sequence.

In a next step S2, the transduced CD34-expressing HSPC are differentiated into immature megakaryocytes, then maturation of megakaryocytes is induced and finally platelets are generated from mature megakaryocytes.

Alternatively, platelets can be generated from transduced mature megakaryocytes derived from transduced (immature) megakaryocytes or from transduced progenitors of megakaryocytes (in particular after differentiation into megakaryocytes and/or maturation of megakaryocytes).

In this embodiment, the platelets express the at least one therapeutic product.

In other words, the platelets contain the at least one therapeutic product.

In an alternative embodiment, the method could comprise more steps.

In this embodiment, the therapeutic sequence comprises a sequence encoding for siRNA.

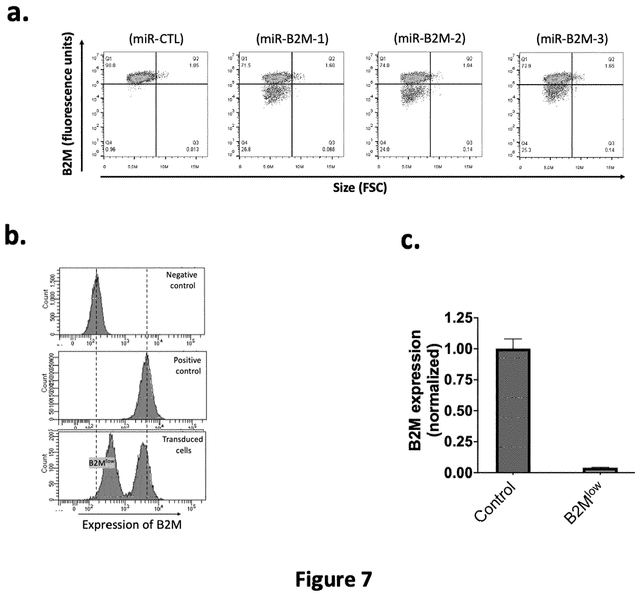

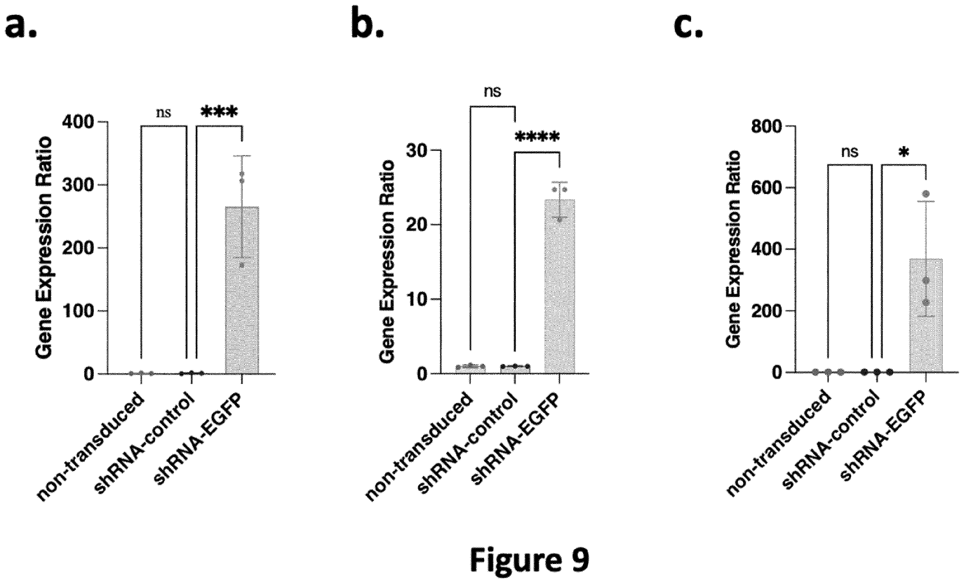

Alternatively, or additionally, the therapeutic sequence could comprise a sequence encoding for shRNA (cf. **Fig. 9****)** and/or miRNA (cf. **Fig. 7****)** and/or mRNA.

In this embodiment, the therapeutic sequence is single.

Alternatively, the therapeutic sequence is in tandem for multiplexing.

In this embodiment, the therapeutic product is a siRNA against podoplanin.

Additionally, or alternatively, the therapeutic product could be a miRNA against podoplanin.

Not shown here is that the DNA-based vector can further comprise at least one coding sequence for a receptor protein, e.g. a cell or tissue or organ specific receptor, wherein the platelets express the receptor protein.

Also not shown is that the receptor protein can be a receptor complex protein.

Alternatively, the receptor protein could be a non-modified receptor protein or a chimeric receptor protein, or a chimeric receptor protein containing a gain-of-function mutation.

Also not shown here is that the coding sequence for the receptor protein can comprise mRNA.

The receptor could be specific for a cell type, a tissue or an organ.

Also not shown in this embodiment is that the at least one coding sequence for the receptor protein can encode for a scFv.

Also not shown in this embodiment is that the coding sequence also can encode for a scFv together with a signaling intracellular domain.

Alternatively, the coding sequence could only encode for a single-chain variable fragment (scFv), a ligand, a receptor, a nanobody, designed ankyrin repeat proteins (DARPins), a chimeric antigen receptor and/or an adapter chimeric antigen receptor (adCAR), in particular together with a transmembrane domain or anchoring region such as a GPI-anchor.

In general, the platelets can be produced for therapeutic use.

Not shown in this embodiment is that a pharmaceutical preparation comprising engineered platelets obtained by a method according to Fig. 1, expressing at least one therapeutic sequence and/or at least one coding sequence for a protein of a receptor, e.g. a cell or tissue or organ specific receptor can be used in a method for the treatment of a disease.

Also not shown is that a pharmaceutical preparation comprising engineered platelets obtained by a method according to Fig. 1, expressing at least one therapeutic sequence and/or at least one coding sequence for a protein of a receptor, e.g. a cell or tissue or organ specific receptor can be used in a method for the treatment of at least one of cancer, acute inflammation, chronic inflammation, bleeding, coagulation, liver disease.

Also not shown is that engineered platelets, obtained by a method according to Fig. 1, expressing at least one therapeutic sequence and/or at least one coding sequence for a receptor protein, e.g. a cell or tissue or organ specific receptor protein, can be used in a method for the treatment of a disease.

Also not shown is that engineered platelets, obtained by a method according to Fig. 1, expressing at least one therapeutic sequence and/or at least one coding sequence for a receptor protein, e.g. a cell or tissue or organ specific receptor protein, can be used in a method for the treatment of at least one of cancer, acute inflammation, chronic inflammation, bleeding, coagulation, liver disease.

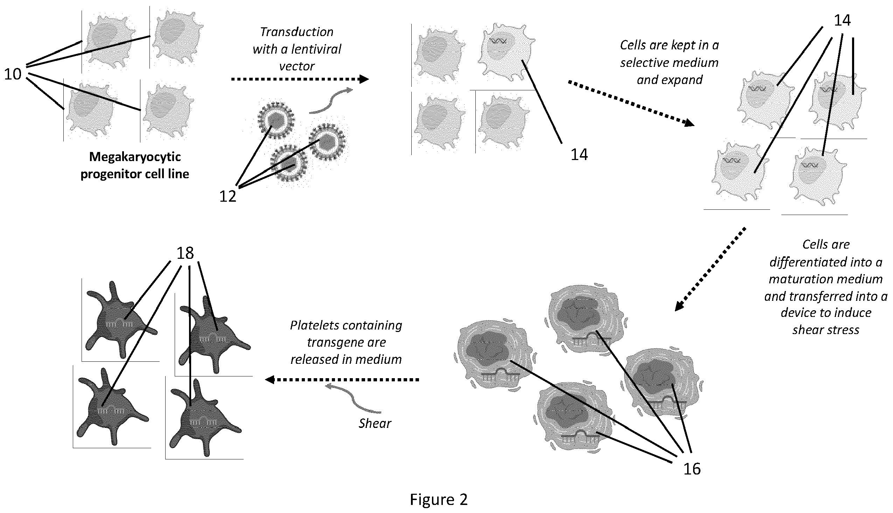

**Fig. 2** illustrates an example of the method according to the present invention for producing engineered platelets.

Megakaryocytic progenitor cells 10 are first transduced with lentiviral particles 12 to express therapeutic compounds (e.g. miRNA against PDPN).

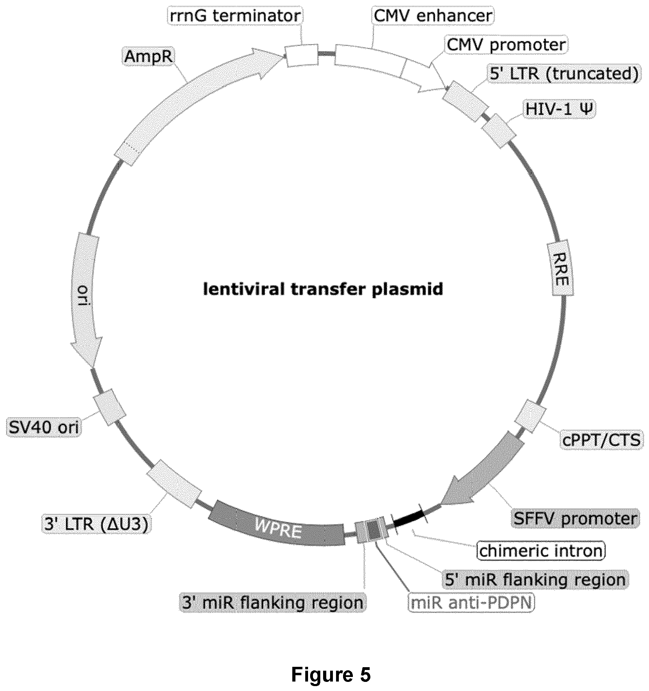

An example of a lentiviral particle, i.e. a lentiviral transfer plasmid is illustrated in Fig. 5.

In particular, the lentiviral particles 12 transduce an expression cassette comprising a therapeutic sequence (e.g. a sequence encoding for miR PDPN).

The therapeutic sequence in this example is a sequence encoding for anti-PDPN miRNA.

Successfully transduced cells 14 are selected.

Cells are then expanded in a selective medium and finally differentiated into mature megakaryocytes 16.

After their transfer into a microfluidic device to generate adhesion and shear, mature megakaryocytes 16 are releasing platelets 18 containing the exogenous therapeutic compound, encoded by the therapeutic sequence.

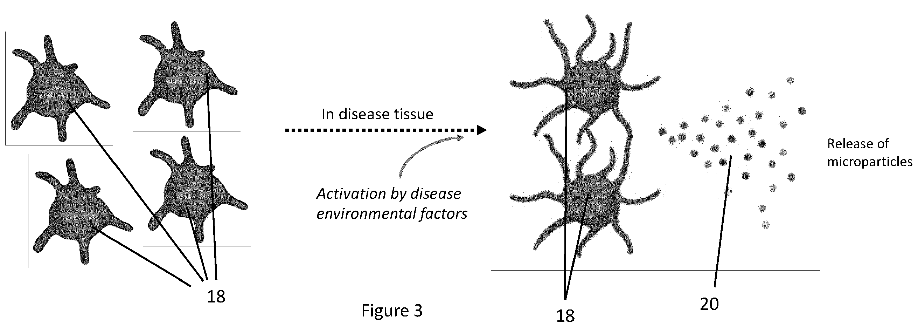

**Fig. 3** illustrates the release of microparticles from platelets.

Shown are platelets 18.

In disease tissue, such as cancer, liver disease, or during bleeding, platelets are activated by environmental factors, such as cytokines, thrombin and/or collagen, and release the content of their granules, as well as microparticles 20.

Platelets 18 produced by the method according to the present invention (c.f. Fig. 1 and/or Fig. 2) express at least one therapeutic sequence and thus, microparticles 20 released from these platelets 18 comprise the respective therapeutic product.

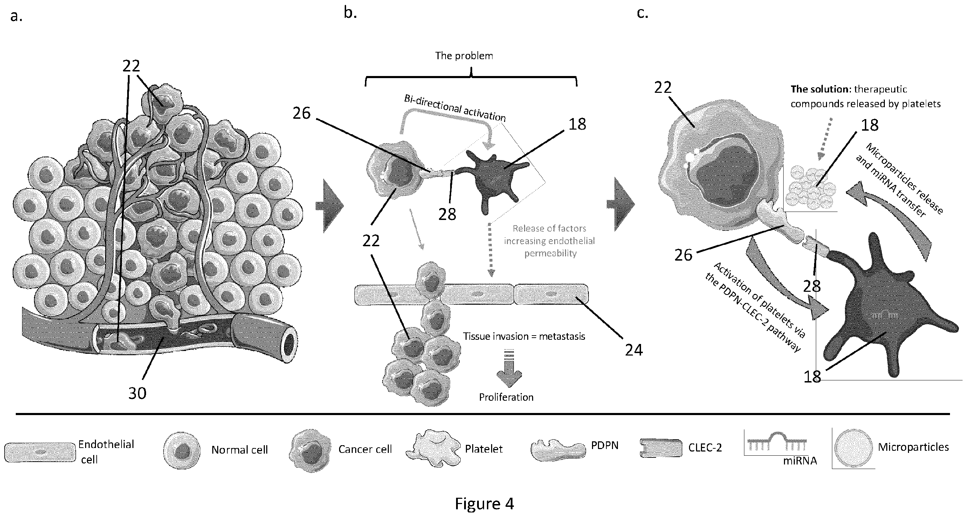

**Fig. 4** shows a schematic view of engineered platelets releasing a therapeutic compound at a site of interest.

Cancer cells 22 in tumors release metastatic cells 22 in the blood stream 30. Further, cancer cells 22 or metastatic cells 22 overexpress PDPN 26.

Platelets 18 naturally express C-type lectin domain family 1 member B (CLEC-2) 28, the receptor for PDPN 26. Here, platelets 18 are engineered to also express miRNA against PDPN (anti PDPN miRNA).

Platelets 18 are activated by metastatic cancer cells 22 in the blood stream 30, by the PDPN-CLEC-2 pathway, releasing microparticles 20 comprising chemokines, cytokines, and factors leading to increased endothelial cell 24 permeability, invasion of cancer cells 22 into tissue, and proliferation. Engineered activated platelets 18 also liberate miRNA (alternatively or additionally siRNA) against PDPN in the vicinity of tumors via microparticles 20.

These microparticles 20 will be engulfed by surrounding cancer or metastatic cells 22, and their PDPN expression will be downregulated. Localized PDPN downregulation can decrease tumor progression and metastasis.

In other words, engineered platelets 18, activated by the PDPN-CLEC-2 pathway, release exogenous therapeutics (here anti-PDPN miRNA), interfering with the disease progression.

If the therapeutic is an anti-PDPN as represented here, it will create a virtuous circle, with less platelet 18 activation and decreased tumor growth.

**Fig. 5** shows an example of a lentiviral transfer plasmid with an expression cassette comprising a therapeutic coding sequence.

In this embodiment, the therapeutic coding sequence encodes for PDPN.

Of importance, the plasmid is showing the spleen focus-forming virus (SFFV) promotor (pSFFV), less susceptible to epigenetic silencing, driving the expression of a transgene, here a miRNA.

The transfer plasmid further comprises the following segments/sequences:
cPPT/CTS segment (short for central polypurine tract/chain termination sequence): this sequence helps to increase the efficiency of transduction.

In this embodiment, the cPPT/CTS segment comprises 118 base pairs.

RRE (short for Rev response element, in particular HIV-1 Rev response element): It allows the nuclear export of viral RNA by the viral Rev protein during viral packaging. In particular, all lentiviruses encode a regulatory protein Rev that is essential for post-transcriptional transport of the unspliced and incompletely spliced viral mRNAs from nuclei to cytoplasm. The Rev protein acts via binding to an RNA structural element known as the Rev responsive element (RRE).

HIV-1 cp: HIV-1 packaging signal required for the packaging of viral RNA into virus.

3'LTR (ΔU3) (short for 3' long terminal repeat that deletes the U3 region): A truncated version of the HIV-1 3' long terminal repeat that deletes the U3 region. This leads to the self-inactivation of the promoter activity of the 5' LTR upon viral vector integration into the host genome (due to the fact that 3' LTR is copied onto 5' LTR during viral integration). The polyadenylation signal contained in 3' LTR-ΔU3 serves to terminate all upstream transcripts produced both during viral packaging and after viral integration into the host genome.

5' LTR (short for 5' long terminal repeat): A deleted version of the HIV-1 5' long terminal repeat. In wildtype lentivirus, 5' LTR and 3' LTR are essentially identical in sequence. They reside on two ends of the viral genome and point in the same direction. Upon viral integration, the 3' LTR sequence is copied onto the 5' LTR. The LTRs carry both promoter and polyadenylation function, such that in wildtype virus, the 5' LTR acts as a promoter to drive the transcription of the viral genome, while the 3' LTR acts as a polyadenylation signal to terminate the upstream transcript. On the present vector, 5' LTR-ΔU3 is deleted for a region that is required for the LTR's promoter activity normally facilitated by the viral transcription factor Tat. This does not affect the production of viral RNA during packaging because the promoter function is supplemented by the CMV promoter engineered upstream of Δ5' LTR.

CMV promoter/enhancer (short for cytomegalovirus major immediate-early (CMV) promotor/enhancer): enables production of viral RNA during packaging to compensate for the partially deleted 5' LTR.

RrnG: Transcription terminator from the E. coli ribosomal RNA rrnG operon.

AmpR (short for ampicillin resistance): Nucleotide sequence for the ampicillin resistance gene. It allows the plasmid to be maintained by ampicillin selection in E. coli.

Ori (short for origin of replication): particular sequence in the vector at which replication is initiated.

SV40 ori (short for Simian Virus 40 origin for replication): particular sequence in the vector at which replication is initiated bidirectionally.

WPRE (short for Woodchuck Hepatitis Virus Posttranscriptional Regulatory Element): it enhances viral RNA stability in packaging cells, leading to higher titer of packaged virus.

3' miR flanking region: a region of DNA that is adjacent to the 3' end of the therapeutic coding sequence (here miR, a sequence encoding for microRNA against PDPN), allows formation of functional pre-miRNA.

5' miR flanking region: a region of DNA that is adjacent to the 5' end of the therapeutic coding sequence (here miR, a sequence encoding for microRNA against PDPN), allows formation of functional pre-miRNA.

Chimeric intron: sequence to significantly increase transgene expression.

miR: sequence for expressing miRNA; therapeutic sequence of the pre-miRNA (could alternatively be e.g. sequence for expressing siRNA). Coding human sequence (5'-3', stem loop-forming precursor RNAs) for miR-29b is CUUCAGGAAGCUGGUUUCAUAUGGUGGUUUAGAUUUAAAUAGUGAUUGUCUAGCACC AUUUGAAAUCAGUGUUCUUGGGGG (SEQ ID NO: 4), and for miR-125a is UGCCAGUCUCUAGGUCCCUGAGACCCUUUAACCUGUGAGGACAUCCAGGGUCACAG GUGAGGUUCUUGGGAGCCUGGCGUCUGGCC (SEQ ID NO: 5), (source: https://www.mirbase.org/).

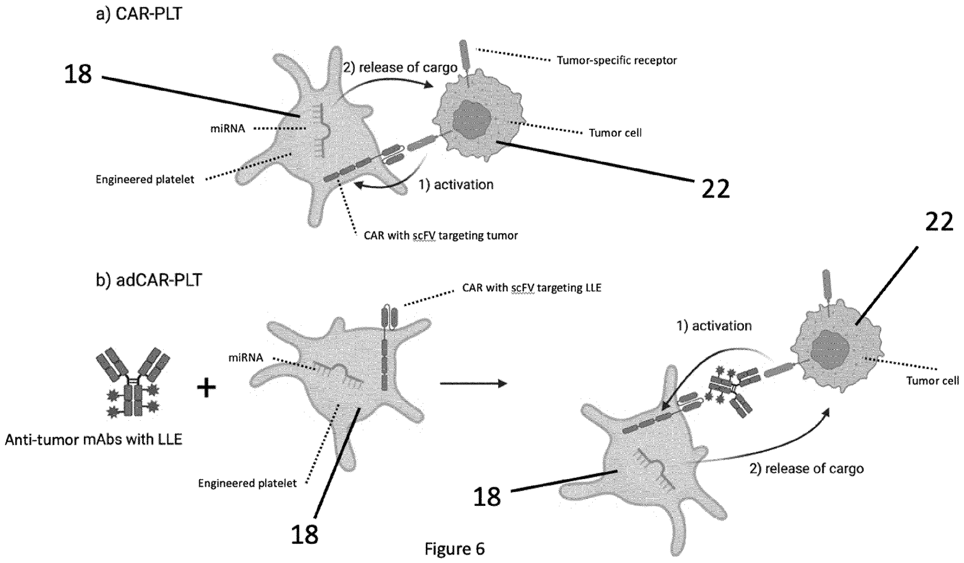

**Fig. 6** shows engineered platelets 18 expressing a chimeric antigen receptor (CAR) (**Fig. 6a**) or an adapter chimeric antigen receptor (adCAR), **(****Fig. 6b****)** activated by cancer cells 22, directly (CAR), or indirectly (adCAR).

The therapeutic strategy using adCAR requires an antibody specific for a target, together with a moiety specific for the adCAR.

**Fig. 7****,** in particular as a prove of principle, shows expression and biological activity of a transgenic miRNA sequence in megakaryocytes.

Three miRNA sequences targeting human beta-2-microglobulin (B2M) (miR-B2M-1, i.e. SEQ ID NO: 6, i.e.
GAAUCUUUGGAGUACGCUGGAUGUUUUGGCCACUGACUGACAUCCAGCGCUCCAAA GAUU;
miR-B2M-2, i.e. SEQ ID NO: 7, i.e.,
   GAAACCUGAAUCUGGAGUACGUUUUGGCCACUGACUGACGUACUCCAGAUUCAGGUU U;
miR-B2M-3, i.e. SEQ ID NO: 8, i.e.,
   GAGUAAGUCAACUUCAAUGUCGGUUUUGGCCACUGACUGACCGACAUUGGUUGACUU ACU);
and one non-targeting control sequence, i.e. miR-CTL, i.e. SEQ ID NO: 9, i.e. GAAAUGUACUGCGCGUGGAGACGUUUUGGCCACUGACUGACGUCUCCACGCAGUAC AUUU) were introduced, in separate experiments, into megakaryocyte progenitors using third generation lentiviral vectors, under the control of a spleen focus-forming virus promoter (pSFFV).

After maturation into megakaryocytes, using a cell culture media based on Iscove's Modified Dulbecco's Medium (IMDM), supplemented with stem cell factor (SCF), interleukin-3 (IL-3) and thrombopoietin (TPO) receptor agonist peptide (Cwirla et al., 1997), expression of B2M has been assessed by flow cytometry (cf. **Fig. 7a****).** All three specific miRNA sequences (i.e. miR-B2M-1, miR-B2M-2, miR-B2M-3) decreased the expression of B2M.

MiR-B2M-1 was chosen for further analysis.

In a next step, MEG-01, a megakaryocyte cell line, was transduced with lentiviral vectors containing miR-B2M-1. After cell sorting to select a population expressing a low level of B2M (defined as B2M^{low}, cf. **Fig. 7b****,** residual expression of 6% compared to control, measured by flow cytometry, using antibody clone 2M2 (official name beta-2-microglobulin, also known as IMD43) (from BioLegend), low level of B2M expression was confirmed (cf. **Fig. 7c****),** a residual expression of 4% compared to control (normalized to Hypoxanthine Phosphoribosyltransferase 1 (HPRT1) expression), by real time qPCR, using primers and internal probes from Integrated DNA Technologies (B2M: Hs.PT.58v.18759587; HPRT1: Hs. PT.58v.4562157).

In a next step, platelets can be generated from the transduced megakaryocytes, the platelets showing low expression of B2M.

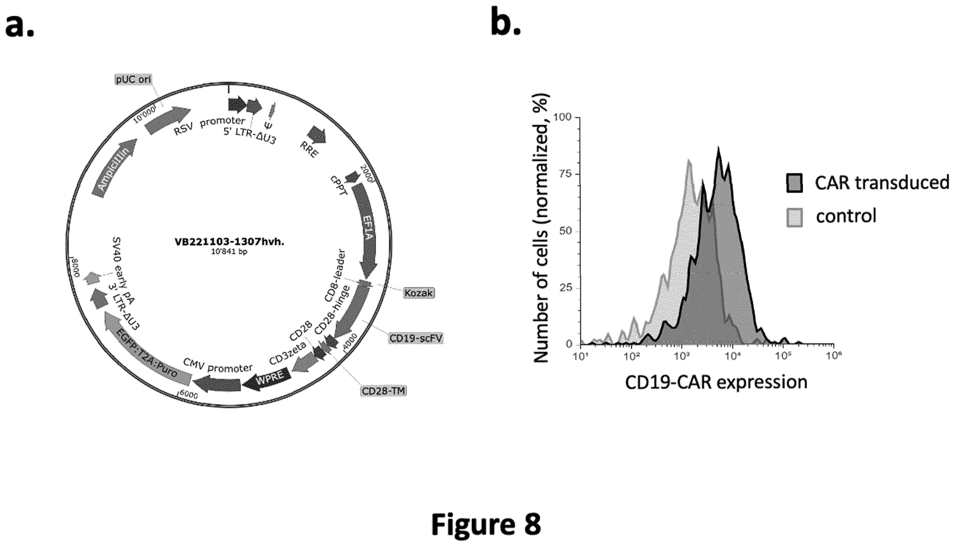

**Fig. 8** shows an example of chimeric antigen receptor (CAR) expression on megakaryocytes. Human CD34⁺ cells have been transduced with a lentiviral vector to express a CAR protein (CD19-scFv/CD28/CD3zeta, expression plasmid for the lentiviral vector shown in **Fig. 8a****).** After 12 days of differentiation towards megakaryocytes in a cell culture media containing TPO receptor agonists peptides, an expression of the CAR at the surface of the megakaryocytes (CD42b⁺) was observed by flow cytometry, using a fusion protein containing the human CD19 extracellular domain and a mutated human IgG1 Fc region (biotinylated CD19-CAR detection Reagent, Miltenyi Biotec), followed by detection with an anti-biotin antibody, labelled with APC-Vio770. **Fig. 8b** shows the expression of a CAR capable to bind CD19.

In a next step, platelets can be generated from the megakaryocytes derived from the transduced CD34⁺ cells, the platelets showing expression of a CAR capable to bind to CD19.

**Fig 9** shows an example of shRNA expressed in platelets and megakaryocytes after transduction of CD34⁺ cells. Human CD34⁺ cells have been transduced with a lentiviral vector to express a shRNA targeting enhanced green fluorescent protein (EGFP) (shRNA-EGFP) (GPP Web Portal - Clone Details, n.d.).

By RT-qPCR and using stem-loop RT primers (Chen et al., 2005), expression of shRNA-EGFP was quantified in CD34⁺ cells (starting cells, cf. **Fig. 9a****),** in CD34⁺-derived megakaryoctes **(****Fig. 9b****),** and in megakaryocyte-derived platelets **(****Fig. 9c****).** ShRNA-EGFP was clearly detected in all cells and in platelets. Statistics: * P<0.05, **P<0.01, *** P<0.001, **** P<0.0001, one-way ANOVA with Sidak correction.

### Bibliography

Burnier, L., Fontana, P., Kwak, B. R., & Anne, A. S. (2009). Cell-derived microparticles in haemostasis and vascular medicine. Thrombosis and Haemostasis, 101(3), 439-451. https://doi.org/10.1160/TH08-08-0521
Chen, C., Ridzon, D. A., Broomer, A. J., Zhou, Z., Lee, D. H., Nguyen, J. T., Barbisin, M., Xu, N. L., Mahuvakar, V. R., Andersen, M. R., Lao, K. Q., Livak, K. J., & Guegler, K. J. (2005). Real-time quantification of microRNAs by stem-loop RT-PCR. Nucleic Acids Research, 33(20), e179-e179. https://doi.org/10.1093/NAR/GNI178
Cortez, M. A., Nicoloso, M. S., Shimizu, M., Rossi, S., Gopisetty, G., Molina, J. R., Carlotti, C., Tirapelli, D., Neder, L., Brassesco, M. S., Scrideli, C. A., Gonzaga Tone, L., Georgescu, M.-M., Zhang, W., Puduvalli, V., & Calin, G. A. (2010). miR-29b and miR-125a Regulate Podoplanin and Suppress Invasion in Glioblastoma HHS Public Access. Genes Chromosomes Cancer, 49(11), 981-990. https://doi.org/10.1002/gcc.20808
Cwirla, S. E., Balasubramanian, P., Duffin, D. J., Wagstrom, C. R., Gates, C. M., Singer, S. C., Davis, A. M., Tansik, R. L., Mattheakis, L. C., Boytos, C. M., Schatz, P. J., Baccanari, D. P., Wrighton, N. C., Barrett, R. W., & Dower, W. J. (1997). Peptide Agonist of the Thrombopoietin Receptor as Potent as the Natural Cytokine. Science, 276(5319), 1696-1699. https://doi.org/10.1126/SCIENCE.276.5319.1696
Gatter, K. C., Brown, G., Strowbridge, I., Woolston, R. E., & Mason, D. Y. (1983). Transferrin receptors in human tissues: their distribution and possible clinical relevance. Journal of Clinical Pathology, 36(5), 539-545. https://doi.org/10.1136/JCP.36.5.539
GPP Web Portal - Clone Details. (n.d.). Retrieved April 12, 2023, from https://portals.broadinstitute.org/gpp/public/clone/details?cloneld=TRCN0000231756
Martin-Villar, E., Borda-D'Agua, B., Carrasco-Ramirez, P., Renart, J., Parsons, M., Quintanilla, M., & Jones, G. E. (2015). Podoplanin mediates ECM degradation by squamous carcinoma cells through control of invadopodia stability. Oncogene, 34(34), 4531. https://doi.org/10.1038/ONC.2014.388
Naito, Y., Yoshimura, J., Morishita, S., & Ui-Tei, K. (2009). SiDirect 2.0: Updated software for designing functional siRNA with reduced seed-dependent off-target effect. BMC Bioinformatics, 10(1), 1-8. https://doi.org/10.1186/1471-2105-10-392/FIGURES/4
Qi, R., Qiao, T., & Zhuang, X. (2016). Small interfering RNA targeting S100A4 sensitizes non-small-cell lung cancer cells (A549) to radiation treatment. OncoTargets and Therapy, 9, 3753-3762. https://doi.org/10.2147/OTT.S106557
Shao, Y., Song, X., Jiang, W., Chen, Y., Ning, Z., Gu, W., & Jiang, J. (2019). MicroRNA-621 Acts as a Tumor Radiosensitizer by Directly Targeting SETDB1 in Hepatocellular Carcinoma. Molecular Therapy, 27(2), 355-364. https://doi.org/10.1016/J.YMTH E.2018.11.005/ATTACH M ENT/7E78EAC7-AA28-409F-8203-FC103D3BD426/MMC1.PDF
Shindo, K., Aishima, S., Ohuchida, K., Fujiwara, K., Fujino, M., Mizuuchi, Y., Hattori, M., Mizumoto, K., Tanaka, M., & Oda, Y. (2013). Podoplanin expression in cancer-associated fibroblasts enhances tumor progression of invasive ductal carcinoma of the pancreas. Molecular Cancer, 12(1), 1-16. https://doi.org/10.1186/1476-4598-12-168/FIGURES/7
Stalker, T. J., Newman, D. K., Ma, P., Wannemacher, K. M., & Brass, L. F. (2012). Platelet Signaling. Handbook of Experimental Pharmacology, 210, 59-85. https://doi.org/10.1007/978-3-642-29423-5_3
Sudimack, J., & Lee, R. J. (2000). Targeted drug delivery via the folate receptor. Advanced Drug Delivery Reviews, 41(2), 147-162. https://doi.org/10.1016/S0169-409X(99)00062-9

### References

- 10: cell line
- 12: vector, in particular lentiviral vector
- 14: clones, successfully transduced cells
- 16: mature megakaryocytes
- 18: platelet, engineered platelet
- 20: microparticles
- 22: cancer cell, metastatic cell
- 24: endothelial cell
- 26: PDPN
- 28: CLEC-2
- 30: bloodstream

- S1: method step 1
- S2: method step 2

## Claims

1. A method for producing engineered platelets, the method comprising at least the following steps:
- transduction of a therapeutic sequence into progenitors of megakaryocytes, CD34-expressing hematopoietic stem and progenitor cells, immature megakaryocytes or megakaryocytes, using a DNA-based vector, the therapeutic sequence encoding at least one therapeutic product,
- generating platelets from mature megakaryocytes derived from the transduced progenitors of megakaryocytes or from the transduced CD34-expressing hematopoietic stem and progenitor cells or from the transduced immature megakaryocytes or from the transduced megakaryocytes,
wherein the platelets contain the at least one therapeutic product.

2. The method of claim 1, **characterized in that** the therapeutic sequence encodes for at least one mRNA, siRNA, shRNA or miRNA.

3. The method of claim 1 or claim 2, **characterized in that** the therapeutic sequence is single or in tandem for multiplexing.

4. The method of claim 2 or claim 3, **characterized in that** the therapeutic product is a miRNA or siRNA against podoplanin (PDPN).

5. The method of any of claims 1 to 4, **characterized in that** the DNA-based vector further comprises at least one coding sequence for a receptor protein, e.g. a cell or tissue or organ specific receptor protein, wherein the platelets express the receptor protein.

6. The method of claim 5, **characterized in that** the receptor protein is a non-modified receptor, a receptor protein complex, a chimeric receptor protein, or a chimeric receptor protein containing a gain-of-function mutation.

7. The method of claim 5 or claim 6, **characterized in that** the coding sequence for the receptor protein comprises mRNA.

8. The method of any of claims 1 to 7, **characterized in that** the platelets are produced for therapeutic use.

9. The use of a pharmaceutical preparation comprising engineered platelets expressing at least one therapeutic sequence and/or at least one coding sequence for a receptor protein, in a method for the treatment of a disease.

10. The use of a pharmaceutical preparation comprising engineered platelets expressing at least one therapeutic sequence and/or at least one coding sequence for a receptor protein, in a method for the treatment of at least one of cancer, acute inflammation, chronic inflammation, bleeding, coagulation, liver disease.

11. The use of engineered platelets expressing at least one therapeutic sequence and/or at least one coding sequence for a receptor protein, in a method for the treatment of a disease.

12. The use of engineered platelets expressing at least one therapeutic sequence and/or at least one coding sequence for a receptor protein, in a method for the treatment of at least one of cancer, acute inflammation, chronic inflammation, bleeding, coagulation, liver disease.

13. A pharmaceutical preparation comprising engineered platelets expressing at least one therapeutic sequence and/or at least one coding sequence for a receptor, e.g. a cell or tissue or organ specific receptor protein, for use in medicine.

14. A pharmaceutical preparation comprising engineered platelets expressing at least one therapeutic sequence and/or at least one coding sequence for a receptor protein, for use in a method for the treatment of at least one of cancer, acute inflammation, chronic inflammation, bleeding, coagulation, liver disease.

15. An engineered platelet expressing at least one therapeutic coding sequence and/or at least one sequence for a receptor protein, for use in medicine.

16. An engineered platelet expressing at least one therapeutic coding sequence and/or at least one sequence for a receptor protein, for use in a method for the treatment of at least one of cancer, acute inflammation, chronic inflammation, bleeding, coagulation, liver disease.
